# NOUVEAU FASCICULE DE BREVET EUROPEEN

(11) **EP 2 050 445 B2**
(45) Date de publication et mention de la décision concernant l'opposition: **21.07.2021**
(45) Mention de la délivrance du brevet: 26.06.2013
(21) Numéro de dépôt: 08021428.1
(22) Date de dépôt: 16.01.1998
(51) Int. Cl.: A61K 31/215, A61K 9/16, A61K 9/20, A61K 9/50, A61K 9/10

(54) **Composition pharmaceutique de fénofibrate présentant une biodisponibilité élevée et son procédé de préparation**
Pharmazeutische Fenofibrat-Zusammensetzung, die eine hohe Bioverfügbarkeit aufweist, und ihr Herstellungsverfahren
Pharmaceutical composition of phenofibrate with high bioavailability and method for preparing same

(30) Priorité: 17.01.1997 FR 9700479
(43) Date de publication de la demande: 22.04.2009
(62) Demande divisionnaire de: 04011004.1
(73) Titulaire: BGP Products Operations GmbH, 6312 Steinhausen (CH)
(72) Inventeur: Stamm, André, 67870 Griesheim (FR); Seth, Pawan, Irvine, CA 92612 (US)
(74) Mandataire: August Debouzy

(56) Documents cités:
- ETHYPHARM: "Réalisation d'une composition à base de fénofibrate micronisé et de polymère hydrophile selon l'inseignement du brevet EP 0 952 829" -, 27 avril 2006 (2006-04-27), pages 1-5, XP002534852
- ETHYPHARM: "Rapport d'essais - Opposition au brevet français 97 00479 (Stamm, Laboratoires Fournier)" -, septembre 2006 (2006-09), XP002534853
- ANONYMOUS: "Mémoire relatif à l'opposition du brevet EP0952829" -, 2 mai 2006 (2006-05-02), XP002534854

## Description

La présente invention a pour objet une nouvelle composition pharmaceutique présentant une biodisponibilité élevée de par une dissolution supérieure et son procédé de préparation. La présente invention concerne plus particulièrement une composition pharmaceutique destinée à une administration par voie orale, contenant un principe actif de faible solubilité aqueuse.

De nombreux principes actifs ont pour inconvénient de présenter une solubilité faible en milieu aqueux, donc de présenter un profil de dissolution insuffisant et par conséquent une faible biodisponibilité dans l'organisme après administration orale. La dose thérapeutique devant être administrée doit donc être augmentée pour obvier cet inconvénient. C'est le cas notamment de nombreux principes actifs hypolipémiants, tels que ceux appartenant à la famille des fibrates.

Le fénofibrate est un hypolipémiant bien connu de la famille des fibrates qui est commercialisé à divers dosages (100 et 300 mg, par exemple Secalip®), mais sous une forme conduisant a une faible biodisponibilité du principe actif. En effet, du fait de sa faible hydrosolubilité, le fénofibrate est mal absorbé au niveau du tube digestif et présente par conséquent une biodisponibilité incomplète, irrégulière et souvent variable d'un individu à l'autre.

Pour améliorer le profil de dissolution du fénofibrate et sa biodisponibilité et réduire ainsi la dose devant être administrée, il serait utile d'augmenter sa dissolution de manière à ce qu'elle puisse atteindre un niveau proche de 100%.

De plus pour le confort du patient, il est avantageux de rechercher une forme galénique ne nécessitant qu'une seule prise par jour qui permette un effet identique à celui obtenu lors de prises multiples.

Un procédé visant à améliorer la biodisponibilité du fénofibrate est décrit dans le brevet EP-A-0 330 532. Ce brevet décrit l'effet de la co-micronisation du fénofibrate avec un tensio-actif, par exemple du laurylsulfate de sodium pour améliorer la solubilité du fénofibrate et augmenter ainsi sa biodisponibilité. Ce brevet enseigne que la co-micronisation du fénofibrate avec un tensioactif solide permet d'améliorer la biodisponibilité du fénofibrate de façon significativement plus importante que l'amélioration que l'on obtiendrait soit par addition d'un agent tensioactif, soit en micronisant uniquement le fénofibrate, soit encore en mélangeant intimement le fénofibrate et le tensioactif micronisés séparément. La méthode de dissolution utilisée est la technique classique de la palette tournante (Pharmacopée Européenne): la cinétique de dissolution du produit est mesurée dans un volume fixe de milieu de dissolution, agité par un dispositif standardisé; un essai a également été réalisé avec une technique alternative de la Pharmacopée Européenne, à savoir la méthode de la cellule à flux continu.

Ce procédé selon le brevet EP-A-0 330 532 conduit à une nouvelle forme galénique où le produit actif, co-micronisé avec un tensioactif solide, présente une dissolution du fénofibrate améliorée, donc une biodisponibilité augmentée, ce qui permet, à efficacité égale, une diminution de la dose quotidienne de médicament: respectivement 67 mg et 200 mg au lieu de 100 mg et 300 mg.

Cependant, le procédé de préparation selon ce brevet n'est pas totalement satisfaisant dans la mesure où il ne conduit pas à une biodisponibilité complète du principe actif et il présente plusieurs inconvénients. La technique de co-micronisation du fénofibrate avec un tensio-actif solide améliore certes la dissolution de ce principe actif, mais cette dissolution reste incomplète.

Il existe donc un besoin pour améliorer la biodisponibilité du fénofibrate afin d'atteindre, dans des temps très courts, un niveau proche de 100% (ou, en tout cas, supérieur aux limites suivantes: 10% en 5 minutes, 20% en 10 minutes, 50% en 20 minutes et 75% en 30 minutes dans un milieu constitué de 1200 ml d'eau additionnée de 2% de Polysorbate 80 ou de 1000 ml d'eau additionnée de lauryl sulfate de sodium 0,025 molaire, avec une vitesse de rotation de la palette de 75 t/min), et ce même lorsque des milieux de dissolution à faible teneur en tensioactif sont utilisés.

La demanderesse a mis en évidence de façon surprenante qu'il est possible de résoudre ce problème par un nouveau procédé de préparation d'une composition pharmaceutique par pulvérisation d'une suspension du principe actif sur un support inerte hydrosoluble. La présente invention concerne également les compositions pharmaceutiques ainsi préparées.

On connaît déjà l'utilisation de polymère tel que la polyvinylpyrrolidone pour la fabrication de comprimés, à des concentrations de l'ordre de 0,5 à 5% en poids, au maximum de 10% en poids. Dans ce cas, la polyvinylpyrrolidone est utilisés comme liant. De même, on connaît l'utilisation de polymère tel que l'hydroxyméthylpropylméthylcellulose comme liant de granulation. Ainsi, EP-A-0 519 144 décrit des pellets d'une substance faiblement soluble, l'oméprazole, qui sont obtenus par pulvérisation sur des pellets inertes, dans un granulateur à lit fluidisé, d'une dispersion ou suspension de principe actif dans une solution contenant ledit polymère. Cependant, là encore, le polymère (HPMC et HIC) n'est utilisé qu'en tant que liant de granulation, en une quantité d'environ 50% en poids du poids du principe actif, ce qui compte tenu de la présence des pellets inertes de grande taille (environ 700µm) et de la quantité totale finale conduit à des teneurs finales en principe actif et en polymère très faibles, de l'ordre de quelque % à peine du poids du pellet final recouvert. Enfin, on remarquera que la dimension des pellets inertes dans ce document est assez élevée, ce qui dans le cas du fénofibrate conduirait à un volume final de la formulation beaucoup trop grand pour une administration aisée par voie orale.

On connaît aussi l'utilisation de polymère tel que la polyvinylpyrrolidone pour la fabrication de "dispersions solides", obtenues en général par co-précipitation, cofusion ou mélange en phase liquide suivie d'un séchage. Il s'agit dans ce cas d'une fixation du principe actif en microparticules isolées sur la polyvinylpyrrolidone, ce qui évite les problèmes de mauvais mouillage du solide et de réagglomération des particules. L'article "Stable Solid Dispersion System Against Humidity", par Kuchiki et al, Yakuzaigaku, 44, No.1, 31-37 (1984) décrit une telle technique de préparation de dispersions solides utilisant de la polyvinylpyrrolidone. Les quantités de PVP sont alors ici très importantes, et les rapports principe actif sur PVP sont compris entre 1/1 et 1/20. Dans ce cas cependant, il n'y a pas de support inerte.

On connaît encore d'après le document WO-A-96 01621 une composition à effet retard, comprenant un noyau inerte (silice dans tous les exemples) revêtu d'une couche comprenant le principe actif en mélange avec un polymère hydrophile, le rapport pondéral principe actif/polymère étant compris entre 10/1 et 1/2 et le rapport pondéral principe actif/noyau inerte étant compris entre 5/1 et 1/2, avec une couche externe pour conférer l'effet retard. Ces compositions peuvent être comprimées. Le polymère hydrophile peut être de la polyvinylpyrrolidone. Ce document décrit aussi un procédé de préparation de cette composition ; par exemple dans un granulateur à lit fluidisé, on pulvérise une dispersion de principe actif dans une solution de polymère sur des noyaux inertes. Ce document n'a trait qu'à des compositions à effet retard, le problème technique à résoudre selon ce document étant la compression sans dommage pour la couche externe conférant l'effet retard.

Cependant, rien dans l'état de la technique n'enseigne ni ne suggère la présente invention.

Ainsi, la présente invention fournit une composition de fénofibrate à libération immédiate comprenant :
(a) un support inerte hydrosoluble recouvert d'au moins une couche Contenant du fénofibrate sous forme micronisée avec une taille inférieure à 20 mm, un polymère hydrophile et éventuellement un tensio-actif; ledit polymère hydrophile représentant au moins 20% en poids du poids de l'élément a); et
(b) éventuellement une ou plusieurs phase(s) ou couche(s) externe(s).

Selon un mode de réalisation, un tensio-actif est présent avec le fénofibrate et le polymère hydrophile.

L'invention fournit aussi une composition selon la revendication 1.

L'invention a encore pour objet un procédé de préparation d'une composition pharmaceutique selon l'invention comprenant les étapes de :
(a) préparation d'une suspension de fénofibrate sous forme micronisée avec une taille inférieure à 20 mm, dans une solution de polymère hydrophile et éventuellement de tensio-actif ;
(b) application de la suspension de l'étape (a) sur un support inerte hydrosoluble;
(c) éventuellement enrobage des granulés ainsi obtenus par une ou plusieurs phase(s) ou couche(s).

L'étape (b) est mise en oeuvre de préférence dans un granulateur à lit fluidisé.

Le procédé peut comporter une étape de compression des produits obtenus à l'étape (b) ou (c), avec ou sans excipients supplémentaires.

L'invention a encore pour objet une suspension de fénofibrate sous forme micronisée avec une taille inférieure à 20 mm, dans une solution de polymère hydrophile et éventuellement de tensio-actif.

La présente invention est décrite plus en détail dans la description qui suit, en référence aux dessins annexés, dans lesquels:
- la figure 1 est une représentation graphique d'une étude comparative du profil de dissolution d'une composition selon la présente invention et de celui du Lipanthyl® 200 M;
- la figure 2 est une représentation graphique d'une étude comparative du profil de dissolution d'une composition selon la présente invention et de celui de produits pharmaceutiques disponibles sur le marché allemand;

On entend, dans le cadre de la présente invention, par l'expression "sous forme micronisée" une substance se trouvant sous une forme particulaire, / la dimension des particules étant inférieure ou égale à environ 20 mm.

Avantageusement, cette dimension est inférieure ou égale à 10mm.

On entend, dans le cadre de la présente invention par "support inerte hydrosoluble" tout excipient, généralement hydrophile, pharmaceutiquement inerte, cristallin ou amorphe, sous une forme particulaire, ne conduisant pas à une réaction chimique dans les conditions opératoires utilisées, et qui est soluble dans un milieu aqueux, notamment en milieu acide gastrique. Des exemples de tels excipients sont les dérivés de sucres, tels que lactose, saccharose, de l'amidon hydrolysé (malto-dextrine), etc.. Des mélanges sont aussi appropriés. La dimension particulaire unitaire du support inerte hydrosoluble peut être par exemple comprise entre 50 et 500 microns.

On entend, dans le cadre de la présente invention par "polymère hydrophile" toute substance de poids moléculaire élevé, (par exemple supérieur à 300) ayant une affinité suffisante pour l'eau pour s'y dissoudre ou y former un gel. Des exemples de tels polymères sont: polyvinylpyrrolidone, poly(alcool vinylique), hydroxypropylcellulose, hydroxyméthylcellulose, hydroxypropylméthylcellulose, gélatine, etc.. Des mélanges de polymères sont aussi appropriés.

Le polymère hydrophile préféré est la polyvinylpyrrolidone (PVP). La PVP utilisée dans le cadre de la présente invention présente par exemple un poids moléculaire compris entre 10 000 et 100 000, de préférence par exemple entre 20 000 et 55 000.

Le terme "tensio-actif" tel qu'utilisé dans le cadre de la présente invention est utilisé dans son sens classique. Tout tensio-actif peut être utilisé, qu'il soit amphotère, non-ionique, cationique ou anionique. Des exemples de tels tensio-actifs sont: sodium lauryl sulfate, monooléate, monolaurate, monopalmitate, monostéarate ou un autre ester de sorbitanne polyoxyéthyléné, dioctylsulfosuccinate de sodium (DOSS), lécithine, alcool stéarylique, alcool cétostéarylique, cholestérol, huile de ricin polyoxyéithylénée, glycérides d'acides gras polyoxyéthylénés, poloxainer®, etc.. Des mélanges de tensio-actifs sont aussi appropriés.

Le tensio-actif préféré est le laurylsulfate de sodium, qui peut être co-micronisé avec le fénofibrate.

Les compositions selon l'invention peuvent en outre contenir tout excipient classiquement utilisé dans le domaine pharmaceutique et chimiquement compatible avec le principe actif, tels que les agents liants, les charges, les pigments, les agents de désintégration, les lubrifiants, les agents mouillants, les tampons, etc. On peut citer à titre d'exemple de tels excipients utilisables dans la présente invention: cellulose microcristalline, lactose, amidon, silice colloïdale, talc, esters de glycérol, stéaryl fumarate de sodium, dioxyde de titane, stéarate de magnésium, acide stéarique, polyvinyl pyrrolidone réticulée (AC DI SOL®), carboxyméthylamidon (Explotab®, Primojel®), hydroxypropyl-cellulose, hydroxyméthylcellulose, hydroxypropylméthylcellulose, gélatine, etc..

On entend par "phase ou couche externe" dans le cadre de la présente invention tout revêtement sur l'élément (a) avec le principe actif (formant un "noyau"). En effet, il peut être intéressant de disposer une ou plusieurs phase(s) ou couche(s) au-dessus du noyau revêtu. L'invention couvre ainsi un noyau unique avec une couche, mais aussi plusieurs noyaux dans une phase comme dans le cas de comprimés formés à partir de "noyaux" mélangés avec une phase. Par "phase ou couche externe" dans le cadre de la présente invention, on n'entend pas les révêtements conférant un effet retard à la composition.

Cette couche externe comprend des excipients classiques. On peut aussi disposer une couche comprenant des adjuvants pour la fabrication de comprimés. Selon ce mode de réalisation, la couche externe comprend un agent de désintégration et par exemple un lubrifiant; les granulés ainsi recouverts et mélangés peuvent alors être facilement comprimés et se désintègrent facilement dans l'eau.

Les compositions selon la présente invention comprennent en général, par rapport au poids total de la composition hors phase ou couche externe, un support inerte hydrosoluble représentant de 10 à 80% en poids, de préférence 20 à 50% en poids, le fénofibrate représentant de 5 à 50% en poids, de préférence 20 à 45% en poids, le polymère hydrophile représentant de 20 à 60% en poids, de préférence 25 à 45% en poids, le tensio-actif représentant de 0 à 10% en poids, de préférence 0,1 à 3% en poids.

La couche ou phase externe, s'il y en a une, peut représenter jusqu'à 80% en poids du poids total, de préférence jusqu'à 50% en poids.

Le polymère hydrophile est présent de préférence en plus de 25% en poids, par rapport au poids de l'élément a).

Le rapport pondéral fénofibrate/polymère hydrophile peut être compris, par exemple entre 1/10 et 4/1, de préférence par exemple entre 1/2 et 2/1.

Quand un tensio-actif est utilisé, le rapport pondéral tensio-actif/polymère hydrophile peut être compris, par exemple entre 1/500 et 1/10, de préférence par exemple entre 1/100 et 5/100.

Selon un mode de réalisation, la composition selon la présente invention se présente sous la forme de comprimés.

Ce comprimé résulte avantageusement de la compression d'éléments (a) (sous forme de granulés) avec une phase externe.

Selon un autre mode de réalisation, la composition selon la présente invention se présente sous la forme de granulés enfermés dans une gélule, par exemple de gélatine, ou dans un sachet.

Les compositions selon la présente invention sont particulièrement appropriées pour l'administration par voie orale des principes actifs.

La composition selon la présente invention est préparée par un nouveau procédé comprenant la pulvérisation sur les noyaux inertes d'une suspension de principe actif sous forme micronisée dans une solution d'un polymère hydrophile et éventuellement de tensio-actif.

Lorsqu'un tensio-actif est présent, le principe actif peut être co-micronisé avec le tensio-actif. On utilise avec avantage la technique selon le document EP-A-0 330 532.

Le procédé selon l'invention consiste à utiliser le principe de la technique de granulation en lit fluidisé, mais avec des produits de départ spécifiques, afin d'aboutir à un profil de dissolution amélioré et ainsi à une biodisponibilité élevée. En particulier, l'invention fait emploi d'une suspension du principe actif micronisé dans une solution d'un polymère hydrophile et éventuellement d'un tensio-actif.

La technique de granulation en lit fluidisé est largement utilisée dans l'industrie pharmaceutique pour préparer des gélules ou des comprimés. De façon classique selon l'art antérieur, une poudre ou un mélange de poudres (principe actif + excipients) est mis en suspension en lit fluidisé dans le granulateur, et une solution contenant un liant et éventuellement un tensio-actif est pulvérisée sur ce lit pour former des granulés. La technique de granulation en lit fluidisé est bien connue de l'homme de l'art qui se rapportera aux ouvrages de référence, par exemple à l'ouvrage "Die Tablette", de Ritschel, Ed. Cantor Aulendorf, pages 211-212.

L'invention, comme il a été indiqué, comprend la pulvérisation sur un support inerte, d'une suspension de principe actif micronisé avec un polymère hydrophile. A l'issue de la granulation, le granulé qui est formé est constitué de cristaux par ex. de lactose, isolés (ou éventuellement agglomérés entre eux par la solution de pulvérisation), et des particules de principe actif et de PVP collés à la surface des cristaux. Le granulé pourrait de même être constitué de cristaux revêtus agglomérés entre eux, voire même d'un tel agglomérat à nouveau revêtu.

Les compositions selon l'invention peuvent aussi être préparées par d'autres procédés, par exemple par pulvérisation de la solution de principe actif micronisé sur le support inerte hydrosoluble.

Les granulés ainsi obtenus peuvent, si cela est souhaité, être enrobé d'une couche externe ou compacté en des comprimés ou former des agglomérats.

La ou les couche(s) externe(s) est(sont) appliquée(s) par des techniques de revêtement classiques, telles que par revêtement dans une cuve ou en lit fluidisé.

Lorsque le granulé obtenu (ultérieurement revêtu ou non) est compacté pour former des comprimés, cette étape peut être mise en oeuvre par toute technique classique appropriée, par exemple sur machine à comprimer alternative ou rotative.

Le produit de départ important est la suspension de principe actif. Cette suspension est préparée par mise en suspension du principe actif micronisé dans une solution, comprenant le polymère hydrophile et éventuellement un agent tensioactif en solution dans un solvant. Si un tensio-actif est utilisé, il est mis en solution dans le solvant (bécher + agitateur magnétique ou agitateur à pales). Ensuite le polymère hydrophile (PVP) est dispersé sous agitation dans la solution précédemment obtenue. Selon la solubilité du polymère, celui-ci se dissout dans la solution ou forme un gel ou une suspension plus ou moins épais(se). Sous agitation toujours, le principe actif micronisé est dispersé en pluie dans la solution ou suspension précédente pour former une suspension homogène. On peut intervertir l'ordre de ces étapes. Le solvant utilisé peut être aqueux ou organique (par exemple éthanol). On utilise par exemple de l'eau déminéralisée. La concentration en principe actif dans la suspension est de 1 à 40% en poids, de préférence 10 à 25%. La concentration en polymère hydrophile dans la suspension est de 5 à 40% en poids, de préférence 10 à 25%. La concentration en tensio-actif dans la suspension est de 0 à 10% en poids, de préférence inférieure à 5%.

L'invention a aussi pour objet cette nouvelle suspension.

Sans vouloir être liée par une théorie, la demanderesse pense que ce nouveau procédé, par l'utilisation d'une suspension du principe actif micronisé dans une solution de polymère hydrophile, permet l'obtention d'une composition nouvelle dans laquelle le principe actif est sous forme non-réagglomérée.

Les exemples suivants illustrent l'invention sans la limiter. Exemple 1 Préparation d'une composition pharmaceutique de fénofibrate selon l'invention.

On prépare une composition contenant en tant qu'élément a) du fénofibrate micronisé, de la Plasdone®, du Capsulac® et du lauryl sulfate de sodium.

Le fénofibrate micronisé présente une dimension particulaire d'environ 5µm, telle que mesurée à l'aide d'un compteur Coulter.

Le Plasdone X25® correspond à.une polyvinylpyrrolidone PVP ISP et le Capsulac 60® (MEGGLE) correspond à un lactose monohydrate à gros cristaux (taille de particules entre 100 et 400 µm).

Le laurylsulfate de sodium (7g) est dissous dans l'eau (eau déminéralisée, 1750g) et le fénofibrate micronisé (350g) est mis en suspension dans le mélange obtenu (par exemple à l'aide d'un agitateur à hélice à 300 t/min, pendant 10 minutes, puis à l'aide d'un agitateur Ultra Turrax à 10 000 t/min, pendant 10 minutes). On ajoute ensuite sous agitation la PVP (350g), l'agitation (agitateur à hélice) étant poursuivie jusqu'à dissolution de cette dernière (30 minutes). L'ensemble est passé sur un tamis (taille 350 µm) pour éliminer d'éventuels agglomérats.

Séparément, le lactose (400g) est mis en suspension dans un granulateur en lit d'air fluidisé (type Glatt® GPCG1 - Top Spray ou équivalent) et on le porte à une température de 40°C.

La suspension de fénofibrate est pulvérisée sur le lactose. Cette étape est réalisée dans les conditions suivantes: pression de pulvérisation: 2,1 bar; débit d'air 70 m³/h, température d'arrivée d'air: 45°C; température de sortie d'air: 33°C; température produit: 34°C; durée de pulvérisation: 3 h.

Le granulé ainsi obtenu peut être mis en gélules ou transformé en comprimés. Toute technique classique appropriée de préparation de telles formulations galéniques peut être utilisée.

Pour la transformation en comprimés, on ajoute à 191g de granulés obtenus (par exemple à l'aide d'un mélangeur type mélangeur-malaxeur, mélangeur planétaire, ou mélangeur par retournement) la phase externe présentant la composition suivante:
- 56g de Polyplasdone XL® (polyvinylpyrrolidane réticulée, ISP, telle que décrite dans la pharmacopée US "USP - NF" sous le nom de crospovidone, MW moyen > 1000000);
- 88g d'Avicel® PH200 (Cellulose microcristalline);
- 3,5g de stéaryl fumarate de sodium (Mendell, U.S.A.); et
- 2g d'Aeroeil® 200 (silice colloïdale).

La polyvinylpyrrolidone réticulée, la cellulose microcristalline, le stéaryl fumarate de sodium et la silice colloïdale sont des agents respectivement de désintégration, liant, lubrifiant et d'écoulement.

L'obtention du comprimé peut s'effectuer sur une machine à comprimer alternative (par exemple Korsch EKO) ou rotative (par ex. Fette Perfecta 2).

On obtient ainsi des comprimés présentant la composition suivante, exprimée en mg:
- élément (a) :

| | |
|---|---|
| Fénofibrate micronisé | 100,0 |
| PVP | 100,0 |
| Lactose | 114,3 |
| Laurylsulfate de sodium | 2,0 |

- phase (ou couche) externe:

| | |
|---|---|
| PVP réticulée | 92,7 |
| Cellulose microcristalline | 145,7 |
| Stéryl fumarate de sodium | 5,8 |
| Silice colloïdale | 3,3 |

### Exemple 2: Dissolution d'une composition selon l'invention et d'une composition selon l'art antérieur.

a) milieu de dissolution et protocole pour la mesure de la dissolution.
   On recherche un milieu de dissolution qui soit discriminant, c'est-à-dire que deux produits ayant des profils de dissolution très différents dans le suc gastrique présenteront des courbes de dissolution très différentes.
   On utilise à cette fin un milieu aqueux contenant un tensio-actif, à savoir le Polysorbate 80 (mono oléate de sorbitanne polyoxyéthyléné). Ce tensio-actif est facilement disponible auprès de plusieurs fournisseurs, fait l'objet d'une monographie dans les pharmacopées, et est aisé à mettre en oeuvre (produit liquide soluble dans l'eau). D'autres tensioactifs comme le lauryl sulfate de sodium peuvent également être utilisés.
   On utilise la méthode de la palette tournante (Pharmacopée Européenne) dans les conditions suivantes: volume du milieu: 1200 ml; température du milieu: 37°C; vitesse de rotation de la palette: 75 t/min; prélèvements: toutes les 2,5 minutes. La détermination de la quantité dissoute est effectuée par spectrophotométrie. Les essais sont répétés à 6 reprises.
b) résultats.

La composition selon l'invention consiste en deux comprimés dosés à 100 mg de fénofibrate environ, préparés selon l'exemple 1.

La composition selon l'art antérieur est du Lipanthyl ® 200 M de Laboratoires Fournier, dosé à 200 mg de fénofibrate (correspondant à des gélules de 200 mg de fénofibrate co-micronisé avec du laurylsulfate de sodium, et renfermant du lactose, de l'amidon prégélatinisé de la polyvinylpyrrolidone réticulée et du stéarate de magnésium, conformément à l'enseignement du brevet EP-A-0 330 532).

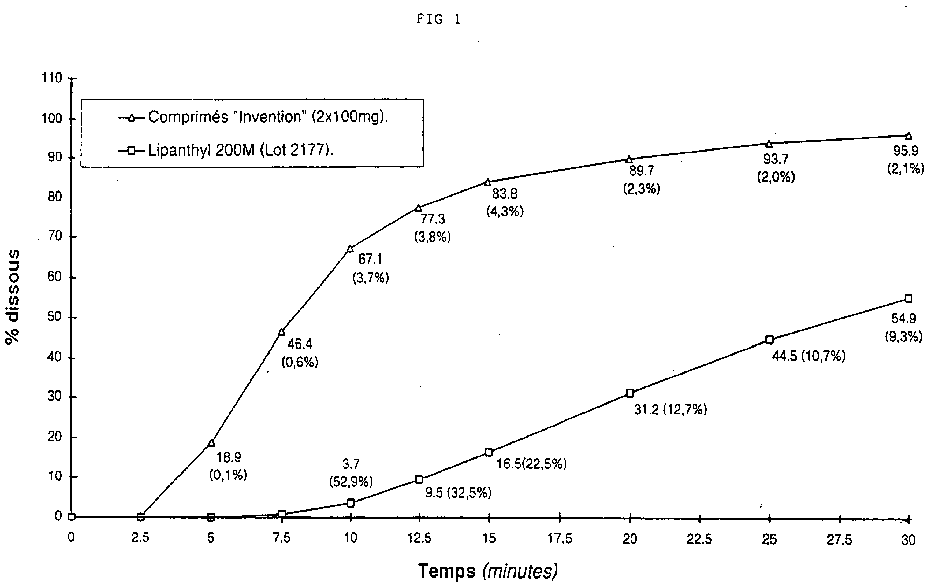

Les résultats obtenus sont représentés graphiquement à la figure 1, sur laquelle sont indiqués le pourcentage de dissolution et entre parenthèses l'écart type observé.

Ces résultats montrent clairement que les compositions selon la présente invention présentent un profil de dissolution nettement supérieur à celui des compositions selon l'art antérieur.

Ces résultats montrent aussi clairement qu'avec les compositions selon l'invention, l'écart type observé est nettement plus faible qu'avec les compositions selon l'art antérieur. Exemple 3: Etude de la biodisponibilité des compositions selon la présente invention et de compositions selon l'art antérieur.

Un essai de biodisponibilité sur volontaires sains a été mené.

Les compositions testées sont les suivantes:
- composition selon l'invention; des gélules contenant les granulés préparés selon l'exemple 1, et dosées à 200 mg de Fénofibrate.
- première composition selon l'art antérieur: Lipanthyl ® 200 M de Laboratoires Fournier, dosé à 200 mg de fénofibrate, identique à celle de l'exemple précédent.
- seconde composition selon l'art antérieur: Secalip ® en gélules (300 mg de fénofibrate sous forme de 3 gélules à 100mg).

L'étude a été réalisée sur 6 volontaires sains recevant une dose unique de fénofibrate, avec une période de repos de 6 jours minimum entre les administrations. Les échantillons pour analyse pharmacocinétique sont recueillis après chaque administration au temps: 0,5; 1 h; 2 h; 3 h; 4 h; 5 h; 6 h; 8 h; 10 h; 12 h; 24 h; 36 h; 48 h; 72 h et 96 heures après la prise du médicament. La teneur en acide fénofibrique dans le plasma est mesurée sur chaque échantillon. Les résultats obtenus sont donnés dans le tableau 1 ci-dessous.

**Tableau 1**

| produit | Dose (mg) | Cmax (µg/ml) | tmax (h) | tl/2 (h) | AUC 0-t (µg.h/ml) | AUC 0-∞ (µg.h/ml) |
|---|---|---|---|---|---|---|
| Invention | 200 | 5,4 | 6 | 23 | 148 | 162 |
| Secalip® 100 | 3 x 100 | 1,1 | 25 | 39 | 53 | 56 |
| Lipanthyl® 200M | 200 | 1,6 | 8,3 | 41 | 71 | 92 |

| | | | | | | |
|---|---|---|---|---|---|---|
| Cmax: Concentration plasmatique maximale tmax: temps nécessaire pour atteindre le Cmax tl/2: Demi vie plasmatique AUC 0- t: Aire Sous la Courbe de 0 à t AUC 0 - ∞: Aire Sous la Courbe de 0 à 1'∞ | | | | | | |

Ces résultats montrent clairement que les compositions selon la présente invention, présentant un profil de dissolution amélioré par rapport aux compositions de l'art antérieur, conduisent à une biodisponibilité du principe actif qui est nettement supérieure à celle obtenue dans le cas des compositions selon l'art antérieur. Exemple 4 Comparaison du profil de dissolution des compositions selon l'invention avec celui de produits actuellement sur le marché en Allemagne Sur le marché allemand on trouve des formulations de fénofibrate à action immédiate ou à action prolongée. Comme en France, les formes à 100 & 300 mg (classiques) coexistent avec des formes à 67 et 200 mg (à biodisponibilité améliorée, selon l'enseignement du brevet EP-A-0 330 532), Ces produits sont les suivants:
- Fénofibrate - Ratiopharm; Ratiopharm - Ulm; Gélules; Composition: Fénofibrate 100 mg; Excipients: Lactose, amidon de mais, stéarate de magnésium, colorant E 171, gélatine.
- Durafenat; Durachemie - Wolfrathausen; Gélules; Composition: Fénofibrate 100 mg; Excipient: Lactose, amidon de maïs, stéarate de magnésium, colorant E 171, gélatine.
- Normalip pro; Knoll- Ludwigshaffen; Gélules; Composition: Fénofibrate 200 mg; Excipients: Crospovidone, gélatine, lactose monohydrate, stéarate de magnésium, amidon de maïs, laurylsulfate de sodium, colorants E 132 et E 171.

On effectue une comparaison entre;
- le comprimé selon l'invention tel que préparé selon l'exemple 1 (2 x 100 mg);
- le Normalip pro ® (200 mg);
- le Lipanthyl ® 200 M (200 mg) (selon l'exemple précédent);
- le Fénofibrate Ratiopharm® (2 x 100 mg);
- le Durafenat ® (2 x 100 mg).

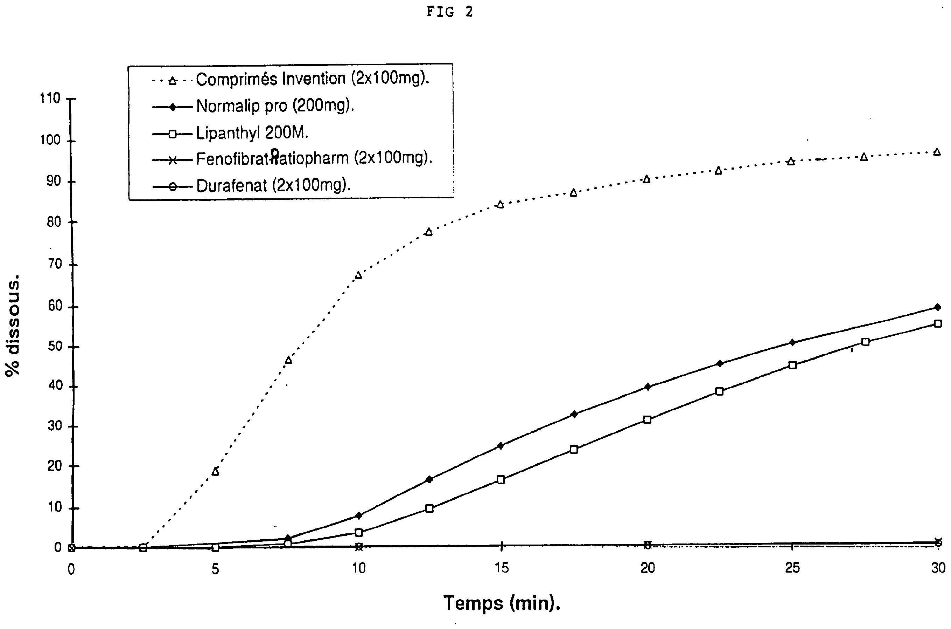

Les tests sont mis en oeuvre dans les mêmes conditions que dans les exemples précédents. Les résultats sont reportés figure 2.

Ces résultats montrent clairement que les compositions selon l'invention présentent une dissolution nettement améliorée par rapport aux compositions selon l'art antérieur.

## Revendications

1. Composition de fénofibrate présentant une dissolution d'au moins 10% en 5 minutes, 20% en 10 minutes, 50% en 20 minutes et 75% en 30 minutes, telle que mesurée conformément à la méthode de la palette tournante à 75 t/min selon la Pharmacopée Européenne, dans un milieu de dissolution constitué de 1200 ml d'eau additionnée de 2% en poids de polysorbate 80.

2. Composition selon la revendication 1, sous forme de comprimé.

3. Composition selon la revendication 1, sous forme de granulés dans une gélule.

## Patentansprüche

1. Fenofibratzusammensetzung, die eine Löslichkeit von mindestens 10% in 5 Minuten, 20% in 10 Minuten, 50% in 20 Minuten und 75% in 30 Minuten aufweist, die unter Verwendung des Blattrührerverfahrens mit 75 Upm gemäß der Europäischen Pharmakopöe in einem Lösungsmedium, das aus 1200 ml Wasser mit 2 Gew.-% Polysorbat 80, gemessen wird.

2. Zusammensetzung gemäß Anspruch 1, in verpreßter Form.

3. Zusammensetzung gemäß Anspruch 1, in Form von Granulaten in einer Kapsel.

## Claims

1. Fenofibrate composition with a dissolution of at least 10% in 5 minutes, 20% in 10 minutes, 50% in 20 minutes and 75% in 30 minutes, as measured in accordance with the rotating blade method at 75 rpm according to the European Pharmacopoeia, in a dissolution medium consisting of 1200 ml of water with 2% by weight of polysorbate 80.

2. Composition according to claim 1, in tablet form.

3. Composition according to claim 1, in granular form in a capsule.
